## Europäisches Patentamt
## European Patent Office
## Office européen des brevets

(11) Publication number: **0 089 145**
**B1**

(12) **EUROPEAN PATENT SPECIFICATION**

(45) Date of publication of patent specification: **16.06.87**

(51) Int. Cl.⁴: **C 07 K 3/06**, C 07 K 15/06, A 61 K 37/12

(21) Application number: **83301135.6**

(22) Date of filing: **03.03.83**

(54) Injectable cross-linked collagen implant material.

(30) Priority: **08.03.82 US 355879**
**06.05.82 US 375665**

(43) Date of publication of application:
**21.09.83 Bulletin 83/38**

(45) Publication of the grant of the patent:
**16.06.87 Bulletin 87/25**

(84) Designated Contracting States:
**AT BE CH DE FR GB IT LI LU NL SE**

(56) References cited:
**EP-A-0 083 868**
**US-A-3 949 073**
**US-A-4 140 537**
**US-A-4 233 360**

(73) Proprietor: **COLLAGEN CORPORATION**
**2500 Faber Place**
**Palo Alto, California 94303 (US)**

(72) Inventor: **Smestad, Thomas L.**
**989 Curtner Avenue**
**San Jose California 95125 (US)**
Inventor: **McPherson, John**
**11012 North Seal Square**
**Cupertino California 95014 (US)**
Inventor: **Wallace, Donald G.**
**387 Hedge Road**
**Menlo Park California 94025 (US)**

(74) Representative: **Harrison, David Christopher et al**
**MEWBURN ELLIS & CO 2/3 Cursitor Street**
**London EC4A 1BQ (GB)**

Courier Press, Leamington Spa, England.

## Description

The invention is in the field of body treating compositions and methods. More particularly it concerns an injectable, cross-linked collagen implant material for augmenting soft tissue in mammals.

Collagen has been used as a paramaceutical carrier, as a surgical prosthesis (sutures and wound dressings), and as an implant material. Chvapil, et al, *Intl. Rev. of Connective Tissue Res.* (1973) 6:1. In many instances the collagen was cross-linked with chemical agents, radiation, or other means to improve its mechanical properties, decrease its immunogenicity, and/or increase its resistance to resorption. This prior cross-linked collagen was solid in nature. Implants made from solid cross-linked collagen were implanted surgically (i.e. were emplaced through incision).

Oliver et at, *Clinical Orthopaedics & Related Research (1976) 115:291—302, Br. J. Exp. Path.* (1980) 61:544—549, and *Conn. Tissue Res.* (1981) 9:59—62, describe implants made by treating skin with trypsin followed by cross-linking with an aldehyde. The resulting solid collagen implants were reported to maintain their original mass after prolonged implantation. A main problem with such solid implants is that they must be implanted surgically. Other disadvantages are that they are not as deformable as injectable implants and residual glutaraldehyde may cause the implant to lose its flexibility due to continuing *in situ* cross-linking.

Schechter, et al, *Br. J. Plas. Surg.* (1975) 28:198—202 disclose glutaraldehyde cross-linked skin that was soaked in L-alanine after cross-linking. The article postulates that the exposure of the skin to L-alanine blocked residual reactive groups of the aldehyde, thereby preventing the release of toxic molecules generated by such groups.

US Patent No. 3949073 describes the use of atelopeptide solutions of collagen as an injectable implant material for augmenting soft tissue. According to the patent, the collagen is reconstituted before implantation and forms a fibrous mass of tissue when implanted. The patent suggests adding particles of insoluble collagen microfibrils to control the shrinkage of the fibrous mass formed at the augmentation site. ZYDERM® collagen implant is a commercial embodiment of the material described in the patent and is composed of reconstituted atelopeptide collagen in saline that contains a small amount of a local anesthetic. While this commerical material is remarkably effective, it may shrink in volume after implantation due primarily to absorption of its fluid component by the body. Thus, if volume constancy, sometimes called "persistency", is essential, an additional injection or injections of supplemental implant material is required.

A principal object of the present invention is to provide a cross-linked collagen implant material that is useful for dermal augmentation and that (1) is uniform ie, it contains only a single physical form of collagen, (2) is injectable, and (3) has improved persistence and resistance to physical deformation relative to ZYDERM® collagen implant.

One aspect of the invention is a novel chemically cross-linked atelopeptide collagen aqueous composition characterized in that (a) it is a fluid at 22°C in the range with a viscosity of 700 to 3000 mPa's as measured on a Nametre model 7.006 viscosimeter at a protein concentration of 20 to 50 mg/ml, and (b) it contains less than 20 ppm residual cross-linking agent.

Another aspect of the invention is a collagen implant material for use in augmenting soft tissue characterized in that it comprises an aqueous dispersion of the above described chemically cross-linked atelopeptide collagen.

Yet another aspect of the invention is a process for preparing the above described chemically cross-linked atelopeptide collagen characterized by (a) reconstituting atelopeptide collagen from an acidic aqueous solution by neutralizing the solution at a reduced temperature and at a hypotonic ionic strength in the range of 0.03 to 0.1, the final pH being in the range 4.9 to 10; (b) cross-linking the reconstituted atelopeptide collagen in an aqueous medium with a cross-linking agent that forms covalent bonds with the collagen to produce a cross-linked collagen; (c) quenching the cross-linking reaction with a quenching agent that reacts with the cross-linking agent; and (d) separating the viscous, cross-linked atelopeptide collagen from the reaction mixture, including washing the collagen to remove other reaction products and unreacted reagents, to yield an aqueous suspension of atelopeptide collagen with a viscosity at 22°C and a protein concentration of 20—50 mg/ml in the range of 700 mPa's to 3000 mPa's, as measured on a Nametre model 7.006 PBD viscosimeter having less than 20 ppm of residual cross-linking agent.

The cross-linked collagen used in the invention may be derived from collagen collected from any number of mammalian sources. The donor need not be genetically similar to the host into which the material is ultimately implanted. Because of their availability, bovine or porcine corium will usually be employed. The first step in making the cross-linked collagen is to prepare atelopeptide collagen in solution from the corium. The animal skin is softened by soaking it is a mild acid and then scraping it to remove hair, epidermis, and fat. The depilated skin is then soaked again in mild acid and then comminuted by grinding, mincing, milling or like physical treatment. The comminution prepares the skin for solubilization.

The divided tissue may be solubilized under nonenaturing conditions my dispersing it in an aqueous acid medium and digesting it with a proteolytic enzyme other than a collagenase. Dilute acid solutions at low temperatures will normally be used to avoid denaturation. Mineral acids such as HCl or carboxylic acids such as acetic, malonic or lactic acids may be used at pHs in the range of about 1.5 to 5 and temperatures of about 5°C to 25°C. A preferred procedure is to disperse the comminuted tissue in HCl to a

2

concentration of 1 to 5 g/l at a pH of about 2 at 20°C. After the tissue is dispersed the enzyme is added and the mixture is incubated to permit the enzyme to digest the telopeptide and other solubilizable components of the tissue. Enzymes that attack the telopeptide portion of the collagen while not denaturing the helical portion are used. Examples of such enzymes are trypsin, pepsin, chymotrypsin, and papain. Pepsin is preferred because it is relatively easily deactivated and removed from the solubilized collagen. The enzyme concentration will usually be in the range of about 0.1% to 10% by weight based on the collagen. The incubation period will typically vary from about two days to two weeks. The progress of the solubilization may be monitored by determining the viscosity of the solution. Once the viscosity reaches a substantially constant level, the solubilization is complete. At this point, the enzyme is deactivated (denatured) and removed.

The enzyme may be deactivated by raising the pH of the solution to at least about 7 by adding an alkaline material such as sodium hydroxide. After the enzyme has been denatured the solution is treated to remove denatured enzyme and the portions of the tissue that were digested during the solubilization. Various dialysis, sedimentation, and filtration techniques may be used to effect such removal. See US Patents 4140537 col. 5 line 48 to col. 6 line 33 and its divisional 4233360 col. 5 line 48 to col. 6 line 33. A preferred procedure is to first lower the pH by adding acid and then clarify the solution by diatomaceous earth sedimentation. The sediment is filtered and the filtrate is concentrated. The concentrate is then fractionated by ion exchange chromatography and further concentrated to produce a substantially pure atelopeptide collagen solution that may be used to make the cross-linked collagen.

The next step in making the cross-linked collagen is to reconstitute the atelopeptide collagen from solution. The reconstitution is preferably done by neutralizing the solution at reduced temperatures, preferably about 10°C to 25°C. The ionic strength of the neutralized solution is preferably hypotonic relative to physiological conditions. Ionic strengths in the range of 0.03 to 0.1, preferably about 0.06, will typically be used. The neutralization involves raising the pH of the solution by adding an appropriate base or buffer, such as $Na_2HPO_4$ or NaOH to a level at which the collagen is solution reaggregates into fibrils. Fiber formation occurs under these conditions at pHs in the range of 4.9 and 10.0. The final pH is preferably in the range of about 5 and 8. Within this range pHs below about 7 favor formation of fine, soft fibrils whereas pHs above about 7 favor formation of coarser fibrils. The softer fibrils are more pliable and form a dispersion that has a creamier texture than dispersions of coarser fibrils. Such texture makes the soft fibril dispersion easier to inject. The duration of the fibril formation step will normally be in the range of about 1/2 to about 18 h. The steps above being similar to those disclosed in EP—A—0083868.

The resulting reconstituted atelopeptide fibrous collagen gel suspension is then cross-linked with a cross-linking agent that forms covalent bonds between itself and the collagen. Usually the agent will be polyfunctional, and more usually bifunctional. The cross-linking conditions are such as to produce a viscous, covalently cross-linked collagen that may be formulated as an injectable fluid and that provides an implant that has improved persistence relative to an implant made from a comparable formulation of non-cross-linked fibrous atelopeptide collagen. When this degree of cross-linking has been reached the cross-linking reaction is quenched by adding a quenching agent. The quenching agent forms an innocuous, water soluble adduct with the cross-linking agent. The concentration of cross-linking agent and the duration of the cross-linking reaction are important process conditions as regards obtaining the degree of cross-linking that provides a viscous, fluid product that is per se injectable. The quenching is important to ensure against further cross-linking and possible changes in product viscosity. The deactivation of reactive cross-linking groups also facilitates the removal of such groups from the product and renders the product less immunogenic relative to nonquenched cross-linked collagens. Aldehydes are preferred cross-linking agents. Examples of aldehydes that may be used to cross-link the collagen are formaldehyde, glutaraldehyde, acetaldehyde, glyoxal pyruvic aldehyde, and dialdehyde starch. Glutaraldehyde is particularly preferred. Compounds that have functional groups that react with the functional groups of the cross-linking agent (eg aldehyde group) to form water soluble adducts may be used to quench the cross-linking reaction. Quenching agents that have free amino groups such as amino acids are preferred. Glycine is particularly preferred. The concentration of glutaraldehyde in the reaction mixture will typically be 0.001% to 0. 05% by weight. The duration of the cross-linking reaction will usually be in the range of one-half hour to about one week. The reaction will normally be carried out at about 10°C to about 35°C. The quenching agent is added in at least stoichiometric proportions relative to the cross-linking agent. Excess quenching agent is preferred.

A particularly preferred cross-linking and quench protocol is: about 0.01% by weight glutaraldehyde for about 16 h at approximately 22°C, quenched with excess glycine to about 0.2 M at approximately 22°C for one to two h.

After the quenching is complete the cross-linked atelopeptide collagen product may be washed with an aqueous buffer solution to remove aldehyde-quenching agent adducts, unreacted aldehyde, aldehyde polymers, and unreacted quenching agent. A sodium phosphate-sodium chloride buffer solution, pH 6.9 to 7.4 is preferred. The washed product may be concentrated, such as by filtration or centrifugation, to a suitable protein concentration range, typically about 20 to 50 mg/ml, preferably about 25 to 40 mg/ml. Protein concentration may be adjusted to this range by addition of buffer or further concentration, as the case may be. The washed product will have a free aldehyde content below about 20 ppm and a viscosity in the range of 700 to 3000 mPa at 22°C measured by an oscillating disk viscosimeter which measures dynamic, not steady flow viscosity. (Nametre Co., model 7.006 PBD).

Final formulation of the aqueous suspension of cross-linked, quenched collagen will typically involve adjusting the ionic strength of the suspension to isotonicity (i.e. 0.15 to 0.2) and adding a local anesthetic, such as lidocaine, to a concentration of about 0.3% by weight to reduce local pain upon injection. The suspension is then loaded into syringes fitted with a #.27 gauge or larger needle for injection. As used herein the term "injectable" means the formulation can be dispensed from such syringes under normal manual pressure. The above described steps in the process for preparing the novel injectable cross-linked collagen are preferably carried out in sterile conditions using sterile materials.

The above described collagen implant material may be injected intradermally or subcutaneously to augment soft tissue, to repair or correct congenital anomalies, acquired defects or cosmetic defects. Examples of such conditions are congenital anomalies such as hemifacial microsomia, malar and zygomatic hypoplasia, unilaterial mammary hypoplasia, pectus excavatum, pectoralis agenesis (Poland's anomaly), and velopharyngeal incompetence secondary to cleft palate repair or submucous cleft palate (as a retropharyngeal implant); acquired defects (post traumatic, post surgical, post infectious) such as depressed scars, subcutaneous atrophy (eg, secondary to discoid lupis erythematosis), enophthalmos in the enucleated eye (also superior sulcus syndrome), acne pitting of the face, linear scleroderma with subcutaneous atrophy, saddle-nose deformity, Romberg's disease and unilateral vocal cord paralysis; and cosmetic defects such as glabellar frown lines, deep nasolabial creases, circum-oral geographical wrinkles, sunken cheeks and mammary hypoplasia.

The following examples illustrate the viscous cross-linked collagen, implant materials made therefrom, the method by which the materials are used, and the merits of implants made of these materials. These examples are not intended to limit the invention in any manner.

Preparation of Atelopeptide Bovine Collagen Solution

Bovine hide was softened and depilated by treatment with HCl. The hide was then comminuted and dispersed in HCl, pH 2, at 8—11 g/l. Pepsin was added to the dispersion at 0.1% by weight based on total protein and the mixture was allowed to incubate for about 100—300h at 15°C to 20°C. NaOH was then added to raise the pH of the incubation medium to about 7 and thereby terminate the digestion. The denatured enzyme was removed from the reaction mixture by sedimentation at reduced pH. The solution was then purified and concentrated by filtration and chromatography to form a 3 mg/ml solution of atelopeptide bovine collagen in dilute aqueous HCl, pH 1—4. This solution is hereinafter referred to as CIS.

Reconstitution of Fibrous Collagen from CIS

Fibrous collagen was reconstituted from CIS by adding 0.02 M $Na_2HPO_4$ to the CIS at 18°C to 20°C to increase its pH to 7.4 ± 0.2 or 5.8 to 6.5. Fibers were allowed to form for 1—2 h.

Preparation of Cross-linked Viscous Collagen
A. Using Collagen Reconstituted at pH 7.4 ± 0.2.

To one hundred-sixty ml of the neutral reconstituted fibrous collagen suspension was added 1.62 ml of 1.0% aqueous glutaraldehyde at pH 3. The glutaraldehyde solution was added gradually with stirring to attain a final level of 0.01%. After a reaction period of 16 h the reaction was quenched by adding 3 M glycine to 0.2 M. The quench time was one hr. The cross-linked collagen was then washed three times with approximately 100 ml of buffer, 0.02 M $Na_2HPO_4$, 0.13 M NaCl, pH 7.4, with centrifuging at 17000 × g for 5 to 7 minutes between each wash. The dynamic viscosity of the collagen was measured by an oscillating disc device (Nametre Co., model 7.006 PBD, measurement at a shear rate of about 5000 $sec^{-1}$) and found to be approximately 700 mPa at 22°C. After the final wash and centrifugation the collagen was resuspended in the buffer to a protein concentration of about 28—35 mg/ml. This dispersion was loaded into syringes fitted with a #27 gauge needle. This collagen preparation is hereinafter designated preparation C.

Preparations of injectable cross-linked collagen fluid were carried out as above using different cross-linking reaction times and glutaraldehyde concentrations. These preparations are listed below.

| Preparation Designation | % Glutaral-dehyde | Reaction Time (h) |
|---|---|---|
| A | 0.01 | 1 |
| E | 0.05 | 1 |
| G | 0.05 | 16 |

B. Using Collagen Reconstituted at pH 5.8 to 6.5.

One % aqueous glutaraldehyde, pH3, was added to the reconstituted fibrous collagen dispersion gradually with stirring to give a final glutaraldehyde concentration of 0.005%—0.010%. Cross-linking was allowed to proceed with stirring for 16 h. The reaction was then quenched by adding 2M glycine with stirring to a final concentration of 0.2 to 0.3M. Quenching continued with stirring for 2—3 h. The quenched, cross-linked collagen was then centrifuged at 16,000 × g for 5 to 10 min, harvested, and resuspended in 10 to 40 vol of 0.02M $Na_2HPO_4$, 0.13M NaCl, pH7.4. This suspension was centrifuged at 16,000 × g for 5 to 20 minutes and the final viscous cross-linked collagen product was harvested. After the final centrifugation the protein concentration was 45 ± 5 mg/ml.

In Vivo Testing of Collagen Preparations

Sprague-Dawley female rats 45—50 days old weighing 120 ± 20g were used as implant recipients.

Groups of three rats were implanted with one of preparations A, C, E and G and with ZYDERM® collagen implant as a control material. Each animal was implanted in two sites: the cross-linked collagen preparation in the right dorsal cranial region; and the control material in the left dorsal cranial region. Approximately one ml of material per site was injected into the subcutaneum.

All materials were explanted in the fifteenth day post implantation. Host tissues were carefully dissected from the explants, and the wet weights were recorded. The percent weight recovery (persistence) was then calculated from the weight implanted. Weighed specimens were then embedded, sectioned, and stained for histological examination. Stains used included hematoxylin, eosin, and Gomori trichrome.

Results of Testing

Summaries of the hitological examinations of preparations A, C, E and G follow:

Preparation A:

This material presented a fairly uniform lacey appearance as compared to implants of more highly cross-linked non-glycine quenched preparations. The latter formulations were generally organized into large densely packed segments with intervening clefts. Clefts also occurred within the preparation A implants but they were smaller and fewer in number. Fibroblast infiltration was excellent throughout the substance of the preparation A implants as well as within the small intervening clefts. New collagen synthesis appeared to be occurring within the clefts. Very few round cells were observed. Vascular channels were good to moderate in the peripheral one-half of the preparation A implants and were not limited to zones of new collagen synthesis. No evidence of encapsulation was observed. Epithelioid cells and multinucleates were absent.

Preparation C:

This material was even more lacey and porous than preparation A. It showed excellent diffuse cell infiltration along with areas of new collagen synthesis. These characteristics, along with the general paucity of round cells make this material the best of the four preparations from a histological viewpoint.

Preparation E:

This preparation was generally less lacey than those cross-linked with 0.01% glutaraldehyde. Fibroblasts were diffusely distributed but fewer in numbers and vascularization was generally limited to the peripheral one-third of the implants. Areas of new collagen synthesis were also observed less frequently.

Preparation G:

This preparation exhibited the most uniform lacey porous appearance of all the materials cross-linked with 0.05% glutaraldehyde. Although cell migration into this material was good, focal areas of multinucleates and an increase in the number of round cells were also apparent; this had not been observed in preparations cross-linked with 0.01% glutaraldehyde.

The following table reports the results of the persistence (percent wet weight recovery of carefully dissected explanted material relative to wet weight of implant) evaluations of the preparations and the control materials.

| Preparation | Persistence |
|---|---|
| A | 77% |
| C | 68 ± 5% |
| E | 82 ± 2% |
| G | 64 ± 5% |
| Control (avg) | 30—40% |

5

**Claims**

1. An aqueous composition of chemically cross-linked atelopeptide collagen characterized in that:
(a) it is a fluid with a viscosity at 22°C in the range of 700 mPa's to 3000 mPa's as measured on a Nametre model 7.006 viscosimeter at a protein concentration of 20 to 50 mg/ml, and
(b) it contains less than 20 ppm of residual cross-linking agent.
2. The collagen of claim 1 wherein the cross-linking agent is glutaraldehyde.
3. A process for preparing a chemically cross-linked atelopeptide collagen charcterized by:
(a) reconstituting atelopeptide collagen from an acidic aqueous solution by neutralizing the solution at a reduced temperature and at a hypotonic ionic strength in the range of 0.03 to 0.1, the final pH being in the range 4.9 to 10;
(b) cross-linking the reconstituted atelopeptide collagen in an aqueous medium with a cross-linking agent that forms covalent bonds with the collagen to produce a cross-linked collagen;
(c) quenching the cross-linking reaction with a quenching agent that reacts with the cross-linking agent; and
(d) separating the viscous, cross-linked atelopeptide collagen from the reaction mixture, including washing the collagen to remove other reaction products and unreacted reagents, to yield an aqueous suspension of atelopeptide collagen with a viscosity at 22°C and a protein concentration of 20—50 mg/ml in the range of 700 mPa's to 3000 mPa's, as measured on a Nametre model 7.006 PBD viscosimeter and having less than 20 ppm of residual cross-linking agent.
4. The process of claim 3 wherein the cross-linking agent is an aldehyde.
5. The process of claim 3 or claim 4 wherein the quenching agent is an amino acid.
6. The process of claim 4 or claim 5 wherein the temperature of step (a) is about 10°C to about 25°C, the cross-linking agent is glutaraldehyde and the concentration of glutaraldehyde in the cross-linking reaction mixture is about 0.05% or below by weight.
7. The process of claim 4 or claim 5 wherein the collagen is bovin corium collagen; the temperature of step (a) is about 10°C to about 25°C; the final pH of step (a) is between 5 to 8; the cross-linking agent is glutaraldehyde; the concentration of glutaraldehyde in the cross-linking reaction mixture is about 0.05% or below by weight; the cross-linking reaction is carried out for about one-half hour to about one week; and the quenching agent is glycine.
8. A collagen implant material for use in augmenting soft tissue in mammals characterized in that it comprises the composition as claimed in claim 1 or claim 2 or the product of the method as claimed in claims 3 to 7, optionally after adjustment of the protein concentration.
9. An implant material according to claim 8 which include also a local anaesthetic.

**Patentansprüche**

1. Eine wässerige Zusammensetzung aus chemisch vernetztem Atelopeptidkollagen, dadurch gekennzeichnet,
(a) daß sie ein Fluid mit einer Viskosität bei 22°C in einem Bereich von 700 bis 3000 mPa.s, gemessen mittels Viskosimeter mit der Handelsbezeichnung Nametre Modell 7.006 bei einer Proteinkonzentration von 20 bis 50 mg/ml ist und
(b) weniger als 20 ppm restliches Vernetzungsmittel enthält.
2. Das Kollagen nach Anspruch 1, worin das Vernetzungsmittel Glutaraldehyd ist.
3. Ein Verfahren zur Herstellung eines chemisch vernetzten Atelopeptid-Kollagens, gekennzeichnet durch
(a) Rekonstituierung eines Antelopeptid-Kollagens aus einer sauren, wässerigen Lösung durch Neutralisieren der Lösung bei einer verringerten Temperatur und einer hypotonen Ionenstärke innerhalb eines Bereiches von 0,03 bis 0,1, wobei der endgültige pH-Wert innerhalb eines Bereiches von 4,9 bis 10 liegt;
(b) Vernetzung des rekonstitutierten Atelopeptid-Kollagens in einem wässerigen Medium mit einem Vernetzungsmittel, das kovalente Bindungen mit dem Kollagen bildet, um ein vernetztes Kollagen zu erzeugen;
(c) Abschrecken der Vernetzungsreaktion mit einem Abschreckmittel, das mit dem Vernetzungsmittel reagiert; und
(d) Abtrennen des viskosen, vernetzten Atelopeptid-Kollagens aus dem Reaktionsgemisch, einschließlich Waschen des Kollagens, um andere Reaktionsprodukte und nicht umgesetzte Reaktionsmittel zu entfernen, um eine wässerige Suspension von Atelopeptid-Kollagen mit einer Viskosität bei 22°C und einer Proteinkonzentration von 20 bis 50 mg/ml in einem Bereich von 700 bis 3000 mPa.s, gemessen mittels Viskosimeter mit der Handelsbezeichnung Nametre Modell 7.006 PBD, und weniger als 20 ppm restlichem Vernetzungsmittel zu erhalten.
4. Das Verfahren nach Anspruch 3, worin das Vernetzungsmittel ein Aldehyd ist.
5. Das Verfahren nach Anspruch 3 oder 4, worin das Abschreckmittel eine Aminosäure ist.
6. Das Verfahren nach Anspruch 4 oder 5, worin die Temperatur des Schrittes (a) etwa 10—25°C

6

beträgt, das Vernetzungsmittel Glutaraldehyd ist und die Konzentration an Glutaraldehyd im Vernetzungsreaktionsgemisch etwa 0,05 Gew.-% oder weniger beträgt.

7. Das Verfahren nach Anspruch 4 oder 5, worin das Kollagen Rinder-Corium-Kollagen ist; die Temperatur des Schrittes (a) etwa 10-etwa 25°C beträgt; der endgültige pH-Wert des Schrittes (a) 5—8 beträgt; das Vernetzungsmittel Glutaraldehyd ist; die Konzentration an Glutaraldehyd im Vernetzungsreaktionsgemisch etwa 0,05 Gew.-% oder weniger beträgt; die Vernetzungsreaktion innerhalb eines Zeitraums von etwa einer halben Stunde bis zu etwa einer Woche durchgeführt wird; und das Abschreckmittel Glyzin ist.

8. Ein Kollagenimplantatmaterial für die Verwendung zur Vermehrung von weichem Gewebe bei Säugetieren, dadurch gekennzeichnet, daß es die Zusammensetzung nach Anspruch 1 oder Anspruch 2 oder das Produkt des in einem der Ansprüche 3 bis 7 beanspruchten Verfahrens, gegebenenfalls nach Einstellung der Proteinkonzentration, enthält.

9. Ein Implantatmaterial nach Anspruch 8, das auch ein Lokalanästheltikum enthält.

## Revendications

1. Composition aqueuse d'un collagène atélopeptide chimiquement réticulé caractérisée en ce que:
(a) c'est un fluide ayant une viscosité à 22°C de 700 mPas à 3000 M Pasen mesurant sur un viscosimètre Nametre modèle 7.006 à une concentration en protéine de 20 à 50 mg/ml, et
(b) elle contient moins de 20 ppm d'agent réticulant résiduel.

2. Collagène de la revendication 1 où l'agent réticulant est le glutaraldéhyde.

3. Procédé de préparation d'un collagène atélopeptide chimiquement réticulé caractérisé par:
(a) la reconstitution du collagène atelopeptide d'une solution aqueuse acide par neutralisation de la solution à une température réduite et à une force ionique hypotonique dans la gamme de 0.03 à 0,1, le pH final étant dans la gamme de 4,9 à 10;
(b) la réticulation du collagène atélopeptide reconstitué dans un milieu aqueux avec un agent réticulant qui forme des liaisons covalentes avec le collagène pour produire un collagène réticulé;
(c) extinction de la réaction de réticulation avec un agent d'extinction qui réagit avec l'agent de réticulation; et
(d) la séparation du collagène atélopeptide visqueux et réticulé de mélange réactionnel, comprenant le lavage du collagène pour enlever d'autres produits réactionnels et les réactifs n'ayant pas réagi, pour donner une suspension aqueuse de collagène atélopeptide ayant une viscosité à 22°C et une concentration en protéine de 20—50 mg/ml dans la gamme de 7000 mPa.s à 3000 mPa.S, en mesurant sur un viscosimètre Nametre modèle 7.006 PBD et ayant moins de 20 ppm d'agent réticulant résiduel.

4. Procédé de la revendication 3 où l'agent de réticulation est un aldéhyde.

5. Procédé de la revendication 3 ou la revendication 4 où l'agent d'extinction est un acide aminé.

6. Procédé selon la revendication 4 ou la revendication 5 où la température de l'étape (a) est d'environ 10°C à environ 25°C, l'agent de réticulation est le glutaraldéhyde et la concentration du glutaraldéhyde dans le mélange de la réaction de réticulation est d'environ 0,05% ou moins, en poids.

7. Procédé de la revendication 4 ou de la revendication 5 où la collagène est du collagène de corion de bovin; la température de l'étape (a) est d'environ 10 à environ 25°C; le pH final de l'étape (a) est compris entre 5 et 8; l'agent de réticulation est le glutaraldéhyde; la concentration du glutaraldéhyde dans le mélange de la réaction de réticulation est d'environ 0,05% ou moins, en poids; la réaction de réticulation est effectuée pendant environ une demi-heure à environ une semaine; et l'agent d'extinction est la glycine.

8. Matériau pour implant à base de collagène à utiliser pour augmenter le tissu mou chez les mammifères caractérisé en ce qu'il comprend la composition selon la revendication 1 ou la revendication 2 ou la produit de la méthode selon les revendications 3 à 7, facultativement après ajustement de la concentration en protéine.

9. Matériau pour implant selon la revendication 8 qui contient également un anesthésique local.